# EUROPEAN PATENT SPECIFICATION

(11) **EP 1 564 212 B1**
(45) Date of publication and mention of the grant of the patent: **12.12.2007**
(21) Application number: 03753600.0
(22) Date of filing: 26.09.2003
(51) Int. Cl.: C07D 401/04, C07D 401/14

(54) **PROCESS AND INTERMEDIATES FOR OBTAINING 1-(1H-BENZIMIDAZOL-2-YL)-4-(PYRIMIDIN-2-YLAMINO)PIPERIDINE DERIVATIVES**
VERFAHREN UND ZWISCHENVERBINDUNGEN ZUR GEWINNUNG VON 1-(1H-BENZIMIDAZOL-2-YL)-4-(PYRIMIDIN-2-YLAMINO)PIPERIDIN-DERIVATEN
PROCEDE ET INTERMEDIAIRES POUR L'OBTENTION DE DERIVES DE 1-(1H-BENZIMIDAZOL-2-YL)-4-(PYRIMIDIN-2-YLAMINO)PIPERIDINE

(30) Priority: 02.10.2002 ES 200202251
(43) Date of publication of application: 17.08.2005
(73) Proprietor: Ragactives, S.L., 47151 Boecillo (Valladolid) (ES)
(72) Inventor: LORENTE BONDE-LARSEN, Antonio, E- 47151 Boecillo (Valladolid) (ES); MORO FERNANDEZ, Agustin, E-47151 Boecillo (Valladolid) (ES); MARTIN JUAREZ, Jorge, E-47151 Boecillo (Valladolid) (ES)
(74) Representative: Maldonado Jordan, Julia
(86) International application number: PCT/ES2003/000488
(87) International publication number: WO 2004/031170

(56) References cited:
- EP-A- 0 217 700
- FR-A- 2 666 582

## Description

### FIELD OF THE INVENTION

The invention refers to a process for obtaining 1-(1*H-*benzimidazol-2-yl)-4-(2-aminopyrimidine)piperidine derivatives through synthesis intermediates characterized in that they contain one piperidine with at least one oxygenated substituent at the 4-position and one benzimidazole at the 1-position. The invention also refers to said intermediates.

### BACKGROUND OF THE INVENTION

Mizolastine [2-({1-[1-(4-fluorobenzyl)-1*H*-benzimidazol-2-yl]-piperidin-4-yl}-methylamino)-4(1*H*) pyrimidinone] (1) is a 1-(1*H*-benzimidazol-2-yl)-4-(2-aminopyrimidine)piperidine derivative 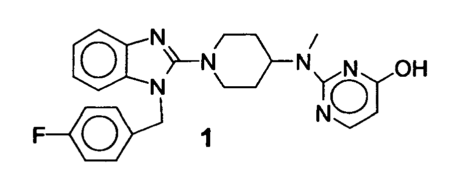 with antihistaminic properties, first disclosed in European patent EP 0 217 700 B1. Said patent discloses several methods (A, B, C and D) for obtaining mizolastine and other similar compounds derived from 1-(1*H*-benzimidazol-2-yl)-4-(2-aminopyrimidine)piperidine, generally based on the condensation of a 2-chlorobenzimidazole with piperidines which are functionalized at the 4-position with nitrogenous groups of the N-H, N-methyl or N-methyl-N-pyrimidinyl type. For the obtainment of these piperidine intermediates it's necessary to resort to more or less elaborated syntheses, using protecting groups for a selective functionalization of the nitrogens in their formation. As an added problem, in the condensation reaction itself with 2-chlorobenzimidazole, it becomes necessary to use other protecting groups (Method A and D), prolonging synthesis, given that in those other cases in which said protecting groups are not used (Method B and C), competition between the nitrogenous groups for condensation with the 2-chlorobenzimidazole occurs, giving rise to mixtures of isomers or else preventing the reaction from satisfactorily taking place.

One of said methods (Method A) comprises reacting 2-chlorobenzimidazole or a derivative thereof, with an (alkoxycarbonyl)aminopiperidine to obtain an intermediate which is subsequently hydrolyzed, and the resulting product is reacted with a pyrimidine. The (alkoxycarbonyl)aminopiperidine used is a prepared and, therefore, expensive raw material, the use of which requires carrying out additional synthesis steps, as well as deprotecting the resulting intermediate after condensation with the 2-chlorobenzimidazole.

Another one of the methods disclosed in said European patent (Method B) comprises reacting 2-chlorobenzimidazole or a derivative thereof, with a 4-aminopiperidine to obtain an intermediate which is reacted with a pyrimidine. When the nitrogenous group present at the 4-position of said 4-aminopiperidine is an amino or methylamino group, the condensation reaction of the 2-chlorobenzimidazole with the aminopiperidine, under the conditions disclosed, takes place very slowly, during a period of 8 days, at a high temperature. Under these reaction conditions, the end product comprises a mixture of isomers, specifically, 15% of the end product is the unwanted isomer, resulting from the alternative condensation reaction of the 2-chlorobenzimidazole with the aminopiperidine through the secondary amine at the 4-position (Tetrahedron Letters, 38(32), 5606-10, 1997).

Another method disclosed in said European patent (Method C) comprises reacting 2-chlorobenzimidazole or a derivative thereof, with a compound resulting from the alkylation of a 1-benzyl- or 1-ethoxycarbonyl- 4-aminopiperidine with a 2-halogen or 2-alkylthiopyrimidine, and subsequently removing the protecting group. Mizolastine can thus be directly obtained. This method has the same previously mentioned drawbacks and, furthermore, the piperidine derivative is a hygroscopic product that is difficult to isolate and purify which, when condensed with the 2-chlorobenzimidazole, generates a number of impurities and a low yield.

The method identified as Method D in said European patent comprises reacting 2-chlorobenzimidazole or a derivative thereof, with an (alkoxycarbonyl)aminopiperidine and, subsequently, with an alkyl halide to obtain an intermediate which is subsequently hydrolyzed, and the resulting product is reacted with a pyrimidine.

Among the pyrimidines used in Methods A, B and D disclosed in said European patent is 2-methylthio-4-hydroxypyrimidine, the use of which releases methylmercaptan in the reaction medium, with the subsequent environmental repercussion. Furthermore, methylmercaptan is a possible contaminant of the end product, especially if the mizolastine is directly obtained as a result of this condensation.

### SUMMARY OF THE INVENTION

The invention confronts the problem of providing an alternative process for obtaining 1-(1*H*-benzimidazol-2-yl)-4-(2-aminopyrimidine)piperidine derivatives, which overcomes all or part of the drawbacks mentioned above.

The solution provided by the invention is based on the fact that the inventors have discovered new synthesis intermediates, 1-[1-(R₁)-1*H*-benzimidazol-2-yl]-4-(R₂) (R₃)-piperidine, wherein R₁ is hydrogen or 4-halobenzyl and one of R₂ or R₃ is optionally protected hydroxy, or else R₂ and R₃, independently from one another, are optionally substituted alkoxy or benzyloxy, or else R₂ and R₃ together form an optionally substituted alkylenedioxy group, characterized by the presence of a piperidine having at least one oxygenated substituent at the 4-position and one benzimidazole group at the 1-position, from which it is surprisingly possible to obtain a 1-(1*H*-benzimidazol-2-yl)-4-(2-aminopyrimidine)piperidine derivative, among them being mizolastine, with a good yield, by condensation with a pyrimidine. Said intermediates come from the condensation between a 2-chlorobenzimidazole (or another equivalent benzimidazole derivative with a leaving group other than the chlorine at the 2-position) and a piperidine with at least one oxygenated substituent at the 4-position, which is a precursor of an amino group characteristic of said 1-(1*H*-benzimidazol-2-yl)-4-(2-aminopyrimidine)piperidine derivatives, but which does not interfere in the condensation reaction between the 2-chlorobenzimidazole and piperidine. The starting materials used for the synthesis of said intermediates are readily available, versatile and economic, enabling their use on an industrial scale.

Said intermediates can be converted by hydrolysis and/or by oxidation in a new intermediate compound, 1-[1-(R₁)-1*H-*benzimidazol-2-yl]-4-piperidone, wherein R₁ is hydrogen or 4-halobenzyl, which, by reductive amination (with optional separation of the intermediate imine formed) provides the corresponding amine which, by reacting with a pyrimidine, allows obtaining said 1-(1*H*-benzimidazol-2-yl)-4-(2-aminopyrimidine)-piperidine derivative. The use of a pyrimidine other than 2-methylthio-4-hydroxypyrimidine allows obtaining an end product which is more environmentally friendly, further preventing the risk of contamination of the end product by methylmercaptan.

Therefore, one aspect of this invention is related to a 1-[1-(R₁)-1*H*-benzimidazol-2-yl]-4-(R₂) (R₃)-piperidine, wherein R₁ is hydrogen or 4-halobenzyl and one of R₂ or R₃ is optionally protected hydroxy, or else R₂ and R₃, independently from one another, are optionally substituted alkoxy or benzyloxy, or else R₂ and R₃ together form an optionally substituted alkylenedioxy group. The process of obtaining said compound constitutes a further aspect of this invention.

Another aspect of this invention is related to a process for obtaining a 1-(1*H*-benzimidazol-2-yl)-4-(2-aminopyrimidine)piperidine derivative, starting from said 1-[1-(R₁)-1*H*-benzimidazol-2-yl]-4-(R₂) (R₃)-piperidine, comprising the hydrolysis and/or oxidation of said compound to obtain a 1-[1-(R₁)-1*H*-benzimidazol-2-yl]-4-piperidone, followed by reductive amination and reaction with a pyrimidine.

Said 1-[1-(R₁)-1*H*-benzimidazol-2-yl]-4-piperidone, wherein R₁ is hydrogen or 4-halobenzyl, and its use in the synthesis of 1-(1*H*-benzimidazol-2-yl)-4-(2-aminopyrimidine)piperidine derivatives, constitute further aspects of this invention.

In another aspect, the invention is related to an imine obtained by reacting said 1-[1-(R₁)-1*H*-benzimidazol-2-yl]-4-piperidone with methylamine, during its reductive amination, and its use in the synthesis of 1-(1*H*-benzimidazol-2-yl)-4-(2-aminopyrimidine)piperidine derivatives.

In another aspect, the invention is related to the use of a 2-chloropyrimidine derivative (or another leaving group other than the chlorine at the 2-position) substituted at the 4-position with a hydroxyl group or precursor thereof, in the condensation with a 1-[1-(R₁)-1*H*-benzimidazol-2-yl]-4-aminopiperidine, coming from the reductive amination of said 1-[1-(R₁)-1*H*-benzimidazol-2-yl]-4-piperidone, for the synthesis of 1-(1*H*-benzimidazol-2-yl)-4-(2-aminopyrimidine)piperidine derivatives.

In another aspect, the invention is related to a process for obtaining mizolastine by hydrolysis of a protected mizolastine derivative, specifically 2-({1-[1-(4-fluorobenzyl)-1*H*-benzimidazol-2-yl]-piperidin-4-yl}-methylamino)-4-benzyloxy-pyrimidine, which constitutes a further aspect of the present invention.

### DETAILED DESCRIPTION OF THE INVENTION

### Compound of Formula I

In one aspect, the invention is related to a 1-[1-(R₁)-1*H*-benzimidazol-2-yl]-4-(R₂) (R₃)-piperidine of Formula I 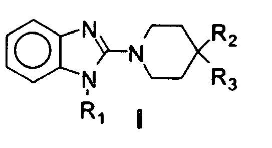 wherein:
R₁ is hydrogen or 4-halobenzyl;
one of R₂ or R₃ is hydrogen and the other one is OH, optionally protected with a protecting group of the hydroxyl group; or
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl,
C₁-C₅ alkoxy and C₁-C₅ hydroxyalkyl.

The term "halo", as it is used in this description, refers to a halogen, preferably fluorine.

The term "C₁-C₅ alkyl" refers to a saturated, linear or branched moiety of a hydrocarbon of 1 to 5 carbon atoms.

The term "C₁-C₅ alkoxy" refers to an -OR group, wherein R is C₁-C₅ alkyl.

The term "C₁-C₅ hydroxyalkyl" refers to a C₁-C₅ alkyl group containing at least one hydroxyl group.

The term "protecting group of the hydroxyl group" includes any group capable of protecting a hydroxyl group, for example, esters, ethers, silyl ethers, ketals, etc.

In a particular embodiment, the compound of Formula I is a compound of Formula Ia, corresponding to a compound of Formula I wherein R₁ is hydrogen or 4-halobenzyl; R₂ is hydrogen; and R₃ is OH.

In another particular embodiment, the compound of Formula I is a compound of Formula Ib, corresponding to a compound of Formula I wherein
R₁ is hydrogen or 4-halobenzyl; and
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy or C₁-C₅ hydroxyalkyl.

In another particular embodiment, the compound of Formula I is a compound of Formula Ic, corresponding to a compound of Formula I wherein R₁ is hydrogen or 4-halobenzyl; R₂ is hydrogen; and R₃ is OH protected with a protecting group of the hydroxyl group.

The compound of Formula I is preferably selected from
a compound of Formula I wherein R₁ is hydrogen or 4-fluorobenzyl; R₂ is hydrogen; and R₃ is OH;
a compound of Formula I wherein R₁ is hydrogen or 4-fluorobenzyl; and R₂ and R₃ are methoxy; and
a compound of Formula I wherein R₁ is hydrogen or 4-fluorobenzyl; and R₂ and R₃, taken together, form an ethylenedioxy group.

The following are particularly preferred compounds of Formula I:
8-[1-(4-fluorobenzyl)-1*H*-benzimidazol-2-yl]-1,4-dioxa-8-azaspiro[4,5]decane (2) 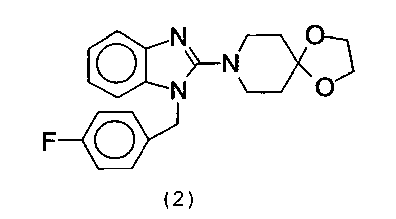
1-[1-(4-fluorobenzyl)-1*H*-benzimidazol-2-yl]-4-hydroxypiperidine (3) 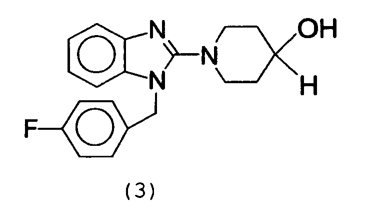 and
1-[1-(9-fluorobenzyl)-1*H*-benzimidazol-2-yl]-4-piperidone dimethylacetal (4)

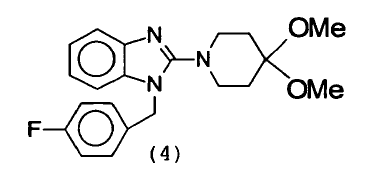

The compound of Formula I can be obtained by means of a process comprising reacting a chlorobenzimidazole of Formula II: 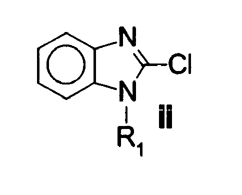
wherein R₁ is hydrogen or 4-halobenzyl;
with a piperidine of Formula III: 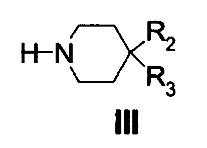 wherein
   one of R₂ or R₃ is hydrogen and the other one is OH, optionally protected with a protecting group of the hydroxyl group; or
   R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
   R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy and C₁-C₅ hydroxyalkyl.

The reaction between the chlorobenzimidazole (II) and piperidine (III) can be carried out in the presence of a solvent and a base, or, alternatively, in the absence of a solvent and in the presence of a base. Although it has been specified for the use of a chlorobenzimidazole (II), a person skilled in the art would understand that another equivalent benzimidazole derivative with a leaving group at the 2-position other than chlorine, can be used, for example, bromine.

In a particular embodiment, the reaction between the chlorobenzimidazole (II) and the piperidine (III) is carried out in an organic solvent with a high boiling point and a base. Virtually any organic solvent with a high boiling point, preferably equal to or greater than 100°C, can be used. However, in a particular embodiment, said organic solvent is selected from alcohols with high boiling points, for example, butanol, isoamylic alcohol, etc.; aromatic hydrocarbons with high boiling points, for example, xylene, etc.; amides with high boiling points, for example, N,N-dimethylformamide, etc.; DMSO, etc., and mixtures thereof. The base can be an organic base, for example, triethylamine, diisopropylethylamine, pyridine or mixtures thereof. Said organic base can optionally be accompanied by an inorganic base, for example, potassium carbonate. Under these conditions, the reaction between the chlorobenzimidazole (II) and piperidine (III) can be carried out at a temperature comprised between 100°C and 180°C, preferably between 110°C and 130°C.

Alternatively, in another particular embodiment, the reaction between the chlorobenzimidazole (II) and piperidine (III) is carried out in the absence of a solvent and in the presence of a base. Just as in the previous embodiment, said base can be an organic base, for example, triethylamine, diisopropylethylamine, pyridine or mixtures thereof, which can optionally be accompanied by an inorganic base, for example, potassium carbonate. In this case, the reaction between the chlorobenzimidazole (II) and piperidine (III) is carried out at a temperature comprised between 70°C and 160°C, preferably between 80°C and 120°C.

Furthermore, if desired, the compound of Formula I wherein R₁ is hydrogen can be converted into a compound of Formula I wherein R₁ is 4-halobenzyl, for example, 4-fluorobenzyl, by reacting with a 4-halobenzyl halide, for example, 4-fluorobenzyl chloride.

The compound of Formula I can be used in the synthesis of 1-(1*H*-benzimidazol-2-yl)-4-(2-aminopyrimidine)piperidine derivatives of Formula IV (shown below), among them being mizolastine.

### Obtaining a 1-(1H-benzimidazol-2-yl)-4-(2-aminopyrimidine)piperidine derivative of Formula IV

In another aspect, the invention is related to a process for obtaining a 1-(1*H*-benzimidazol-2-yl)-4-(2-aminopyrimidine)piperidine derivative of Formula IV 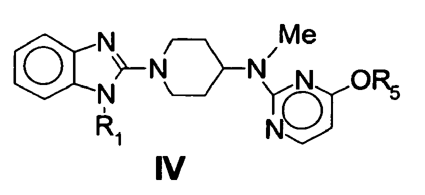 wherein:
R₁ is hydrogen or 4-halobenzyl; and
R₅ is hydrogen; C₁-C₅ alkyl optionally substituted with one or more substituents selected from halogen, nitro and an -OR₆ group, wherein R₆ is C₁-C₅ alkyl or benzyl; or benzyl optionally substituted in the aromatic ring with one or more substituents selected from halogen, nitro and an -OR₆ group, wherein R₆ is C₁-C₅ alkyl or benzyl;
comprising
a) converting a compound of Formula I 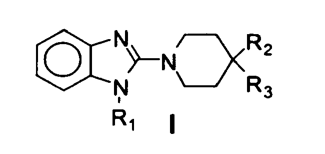 wherein:
   R₁ is hydrogen or 4-halobenzyl; and one of R₂ or R₃ is hydrogen and the other one is OH, optionally protected with a protecting group of the hydroxyl group; or
   R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
   R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy and C₁-C₅ hydroxyalkyl,
   into a 1-[1-(R₁)-1*H*-benzimidazol-2-yl)-4-piperidone of Formula V 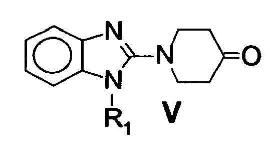 wherein R₁ has the previously mentioned meanings,
   by hydrolysis and/or by oxidation of said compound of Formula I;
b) converting said compound of Formula V into a compound of Formula VI 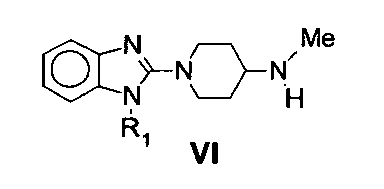 wherein R₁ has the previously mentioned meanings;
   by reductive amination of said compound of Formula V with methylamine and reducing agent, with optional isolation of the intermediate imine formed;
c) reacting said compound of Formula VI with a compound of Formula VII 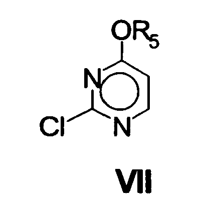 wherein R₅ has the previously mentioned meaning; to obtain a compound of Formula IV; and, optionally
d) converting a compound of Formula IV wherein R₅ is different from hydrogen into a compound of Formula IV wherein R₅ is hydrogen.

### Step a): Conversion of a compound of Formula I into a compound of Formula V

The compound of Formula I can be converted into a compound of Formula V by conventional methods, depending on the functional group present at the 4-position of the piperidine (hydroxyl, protected hydroxyl, hemiacetal or acetal, optionally cyclic), comprising hydrolysis, oxidation or hydrolysis and subsequent oxidation of the hydroxylated intermediate, of said functional group to obtain a carbonyl group at the 4-position of the piperidine.

Therefore, the process for converting the compound of Formula I into the compound of Formula V depends on the compound of Formula I used as the starting material.

In a first alternative (Method A), the compound of Formula I used as the starting material is a compound of Formula Ia, corresponding to a compound of Formula I wherein R₁ is hydrogen or 4-halobenzyl; R₂ is hydrogen; and R₃ is OH. In this case, the compound of Formula V can be obtained by oxidation of the compound of Formula Ia.

Oxidation of the compound of Formula Ia can be carried out by means of any conventional method, based on the use, for example, of NaOCl (Anelli P.L., Montanari F., J. Org. Chem., 52, 2559-62, 1987), chromium (VI) oxides in acetic acid (Fieser, L.F.; Szmuszkovicz, J., JACS, 70, 3352, 1948), Swern reactions with DMSO/(COCl)₂ or the like *(*Chem. Review, 67, 247, 1967), or with any other suitable oxidizing agent capable of oxidizing a hydroxyl group to a carbonyl group.

In a particular embodiment, the oxidation of the compound of Formula Ia is preferably carried out using a combination of oxalyl chloride, dimethylsulfoxide (DMSO) and an organic base, such as triethylamine, diisopropylethylamine, pyridine, etc., in the so-called Swern oxidation, using as a solvent a halogenated solvent, for example, dichloromethane, 1,2-dichloroethane, etc., an aromatic solvent, for example, toluene, etc., and generally, any suitable solvent.

The oxidation of the compound of Formula Ia can be carried out at a temperature comprised between -100°C and 50°C, preferably between -60°C and 0°C.

In a second alternative (Method B), the compound of Formula I used as the starting material is a compound of Formula Ib, corresponding to a compound of Formula I wherein
R₁ is hydrogen or 4-halobenzyl; and
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy or C₁-C₅ hydroxyalkyl.

In this case, the compound of Formula V can be obtained by subjecting said compound of Formula Ib to an acid hydrolysis process by means of the use of an acid capable of hydrolyzing an acetal or a hemiacetal. Virtually any acid capable of hydrolyzing an acetal or a hemiacetal can be used, preferably a strong acid selected from a strong organic acid, a strong inorganic acid, and mixtures thereof. However, in a particular embodiment, said strong acid is selected from methanesulfonic acid, trifluoroacetic acid, sulfuric acid, hydrochloric acid, and mixtures thereof.

The acid hydrolysis of the compound of Formula Ib can be carried out in an organic aqueous medium comprising water and an organic solvent. In a particular embodiment, said organic solvent is chosen from an ether, for example, tetrahydrofuran, dioxane, etc.; an alcohol, for example, methanol; a nitrile, for example, acetonitrile; and mixtures thereof.

The acid hydrolysis of the compound of Formula Ib can be carried out at a temperature comprised between room temperature (15°C-20°C) and the reflux temperature of the medium.

In a third alternative (Method C), the compound of Formula I used as the starting material is a compound of Formula Ic, corresponding to a compound of Formula I wherein R₁ is hydrogen or 4-halobenzyl; R₂ is hydrogen; and R₃ is OH protected with a protecting group of the hydroxyl group.

In this case, the compound of Formula V can be obtained by subjecting said compound of Formula Ic to a process of hydrolysis and subsequent oxidation of the hydroxylated intermediate generated.

In a particular embodiment, the compound of Formula Ic can be hydrolyzed in a basic medium comprising an aqueous or alcoholic solution of a hydroxide of an alkali metal, for example, an aqueous or methanol solution of sodium hydroxide.

Alternatively, the compound of Formula Ic can be subjected to an acid hydrolysis process by means of the use of an acid capable of hydrolyzing a protecting group of the hydroxyl group, in an organic aqueous medium, at a temperature comprised between room temperature and the reflux temperature of the medium. Virtually any acid capable of hydrolyzing a protecting group of a hydroxyl group can be used, preferably a strong acid selected from a strong organic acid, a strong inorganic acid, and mixtures thereof. However, in a particular embodiment, said strong acid is selected from methanesulfonic acid, trifluoroacetic acid, sulfuric acid, hydrochloric acid, and mixtures thereof. The organic aqueous medium in which the acid hydrolysis of the compound of Formula Ic is carried out comprises water and an organic solvent. In a particular embodiment, said organic solvent is selected from an ether, for example, tetrahydrofuran, dioxane, etc.; an alcohol, for example, methanol; a nitrile, for example, acetonitrile; and mixtures thereof.

After the acid or base hydrolysis of the compound of Formula Ic, a hydroxylated intermediate is obtained which, by oxidation of the OH group, yields the compound of Formula V. The oxidation of said hydroxylated intermediate can be carried out by means of the use of an oxidizing agent capable of oxidizing a hydroxyl group to a carbonyl group by convention methods, as previously mentioned in relation to a compound of Formula Ia.

The compound of Formula V 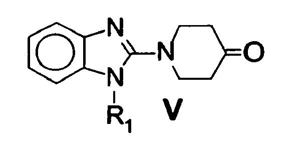
wherein R₁ is hydrogen or 4-halobenzyl,
constitutes a further aspect of this invention. Said compound of Formula V is an intermediate useful in the synthesis of 1-(1*H*-benzimidazol-2-yl)-4-(2-aminopyrimidine)piperidine derivatives of Formula IV. In a particular embodiment, the compound of Formula V is the 1-[1-(4-fluorobenzyl)-1*H-*benzimidazol-2-yl]-4-piperidone compound (5)

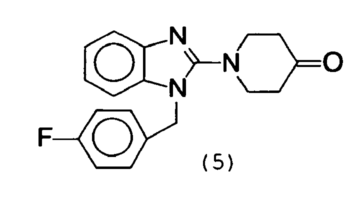

The compound of Formula (5) is useful for obtaining mizolastine.

### Step b): Reductive amination of a compound of Formula V

The reductive amination process of the compound of Formula V can be carried out with or without isolation of the intermediate imine formed.

In a particular embodiment, the process of reductive amination of the compound of Formula V is carried out with the isolation of the intermediate imine formed. In this case, said compound of Formula V is reacted with methylamine, in a protic solvent, such as an alcohol, for example, methanol, to obtain an imine of Formula VIII 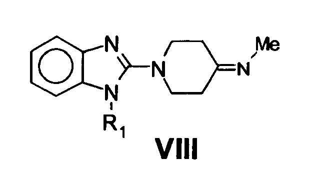 wherein R₁ is hydrogen or 4-halobenzyl.

The imine of Formula VIII can be isolated from the reaction medium by precipitation and separation by conventional methods, for example, filtration. Subsequently, said imine of Formula VIII is subjected to a reduction process by conventional methods to obtain said compound of Formula VI, after dissolving the imine of Formula VIII in the same solvent as the one used for its obtainment or, alternatively, in another suitable solvent, such as tetrahydrofuran, isopropanol, etc. The reduction of the imine of Formula VIII can then be carried out by means of any conventional method. In a particular embodiment, said reduction is carried out by means of the use of a reducing agent, such as, for example, sodium borohydride or sodium cyanoborohydride.

The isolated imine of Formula VIII constitutes a further aspect of this invention and it is an intermediate useful for the synthesis of 1-(1*H*-benzimidazol-2-yl)-4-(2-aminopyrimidine)piperidine derivatives of Formula IV. In a preferred embodiment, said imine of Formula VIII is 1-[1-(4-fluorobenzyl)-1*H*-benzimidazol-2-yl]-N-methyl-4-iminopiperidine (6), useful for the synthesis of mizolastine

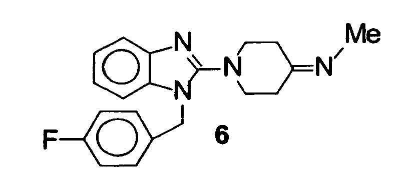

Alternatively, in another particular embodiment, the process of reductive amination of the compound of Formula V is carried out without isolation of the intermediate imine formed VIII. In this case, the compound of Formula V is reacted with methylamine and subsequently with a reducing agent, without isolating the imine, to obtain said compound of Formula VI. The reaction of the compound of Formula V with methylamine and reducing agent is carried out in a suitable solvent, such as a protic solvent, for example, an alcohol, preferably methanol. Virtually any reducing agent can be used for carrying out this process. However, in a particular embodiment, said reducing agent is sodium borohydride or sodium cyanoborohydride.

### Step c): Reaction of a compound of Formula VI with a compound of Formula VII

The reaction between a compound of Formula VI and a compound of Formula VII to obtain a compound of Formula IV is carried out, advantageously, in the presence of an organic base, optionally accompanied by an inorganic base. Virtually any organic base can be used for carrying out said reaction. However, in a particular embodiment, said organic base is selected from triethylamine, diisopropylethylamine, pyridine and mixtures thereof. Alternatively, the reaction medium comprises, in addition to said organic base, an inorganic base. Even though, virtually any inorganic base can be used, in a particular embodiment, said inorganic base is potassium carbonate. The reaction between the compound of Formula VI and the compound of Formula VII can be carried out within a broad range of temperatures, advantageously, between 70°C and 160°C, preferably between 80°C and 120°C.

### Step d): Conversion of a compound of Formula IV wherein R₅ is different from hydrogen into a compound of Formula IV wherein R₅ is hydrogen

In the case of obtaining a compound of Formula IV wherein R₅ is different from hydrogen, for example, a compound of Formula IV with an alkoxy or benzyloxy group, if desired, said compound can be converted into a compound of Formula IV wherein R₅ is hydrogen by conventional methods, for example, by means of hydrolysis in acid medium.

By operating according to the process defined by steps a), b), c) and, optionally d), a 1-(1*H*-benzimidazol-2-yl)-4-(2-aminopyrimidine)piperidine derivative of Formula IV is obtained. In a particular embodiment, the obtained compound of Formula IV is mizolastine [2-({1-[1-(4-fluorobenzyl)-1*H-*benzimidazol-2-yl]-piperidin-4-yl}-methylamino)-4(1*H*)pyrimidinone].

In another preferred embodiment, the obtained compound of Formula IV is 2-({1-[1-(4-fluorobenzyl)-1*H*-benzimidazol-2-yl]-piperidin-4-yl}-methylamino)-4-benzyloxypyrimidine (7)

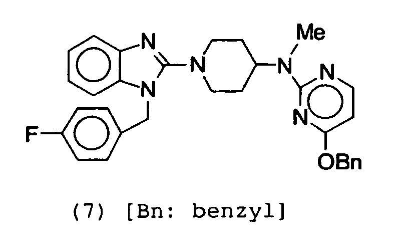

The hydrolysis in acid medium of the 4-benzyloxy group in the compound of Formula (7) allows obtaining mizolastine.

The following examples illustrate the invention and should not be considered to be limiting of the scope thereof.

### EXAMPLE 1

### 8-(1H-Benzimidazol-2-yl)-1,4-dioxa-8-azaspiro[4.5]decane

A mixture of 10 g of 2-chloro-1*H*-benzimidazol, 14 g of 1,4-dioxa-8-azaspiro[4.5]decane and 29 mL of diisopropylethylamine in 70 mL isoamylic alcohol is heated under reflux for 48 hours. Then, it is slowly cooled to room temperature to give a precipitate which is isolated by filtration and washed with 15 mL of cold isoamylic alcohol. The filtered product is resuspended in 100 mL of water for 2 hours, filtered and dried at 60°C, obtaining 12.3 g of 8-(1*H-*benzimidazol-2-yl)-1,4-dioxa-8-azaspiro [4.5] decane. C-NMR (DMSO-d, δ in ppm): 63.70 (CH₂), 106.2 (C), 155.56 (C).

### EXAMPLE 2

### 1-[1-(4-Fluorobenzyl)-1H-benzimidazol-2-yl]-4-hydroxypiperidine (3)

A mixture of 3.9 g of 4-hydroxypiperidine, 5 g of 2-chloro-1-(4-fluorobenzyl)-benzimidazol in 30 mL of isoamylic alcohol is taken to reflux for 12 hours. Once the reaction has finished, the product is isolated by removal of the solvent under reduced pressure and redissolving the residue in 15 mL of ethyl acetate and 15 mL of water. The organic phase is decanted, and the aqueous phase is extracted again with 7 mL of ethyl acetate. The organic phases are brought together and the solvent is removed. The obtained residue is crystallized in toluene, obtaining 3.7 g of 1-[1-(4-fluorobenzyl)-1*H-*benzimidazol-2-yl]-4-hydroxypiperidine (3).
¹³C-NMR (CDCl₃, δ in ppm): 34.17 (CH₂), 47.05 (CH₂), 48.64 (CH₂), 67.04 (CH), 109.27 (CH), 143.40 (C), 163.37 (C).

### EXAMPLE 3

### 8-[1-(4-Fluorobenzyl)-1H-benzimidazol-2-yl]-1,4-dioxa-8-azaspiro[4.5]decane (2)

3.7 g of 8-(1*H*-benzimidazol-2-yl)-1,4-dioxa-8-azaspiro[4.5]decane are slowly added to a mixture of 1.6 g of potassium tert-butoxide in 25 mL of N,N-dimethylformamide, and, after half an hour, 2 mL of 4-fluorobenzyl bromide are slowly added drop-wise. When the reaction is finished, 74 mL of water are very slowly added, and the obtained precipitate is stirred for 2 hours and filtered, obtaining 3.5 g of crude 8-[1-(4-fluorobenzyl)-1*H*-benzimidazol-2-yl]-1,4-dioxa-8-azaspiro[4.5]decane (2)
¹³C-NMR (CDCl₃, δ in ppm): 34.74 (CH₂), 47.14 (CH₂), 64.31 (CH₂), 106.59 (C), 121.94 (CH), 141.70 (C).

### EXAMPLE 4

### 8-[1-(4-Fluorobenzyl)-1H-benzimidazol-2-yl]-1,4-dioxa-8-azaspiro[4.5]decane (2)

A mixture formed by 50 g of 2-chloro-1-(4-fluorobenzyl)benzimidazol, 41 g of 1,4-dioxa-8-azaspiro[4.5]decane and 84 mL of diisopropylethylamine in 350 mL of isoamylic alcohol is heated under reflux for 48 hours. Once the reaction has finished, 175 mL of solvent are distilled and slowly cooled to a temperature of 0-5°C so that a white solid precipitates. The precipitate produced is filtered and washed with 75 mL of cold isoamylic alcohol, and is dissolved again in 250 mL of water and 250 mL of ethyl acetate. It is stirred, and the organic phase is isolated by decanting.

The product is isolated by removing the solvent under reduced pressure, obtaining 60 g of 8-[1-(4-fluorobenzyl)-1*H-*benzimidazol-2-yl]-1,4-dioxa-8-azaspiro[4.5]decane (2).

### EXAMPLE 5

### 1-[1-(4-Fluorobenzyl)-1H-benzimidazol-2-yl]-4-piperidone (5)

A solution of 0.3 mL of oxalyl chloride in 7.5 mL of methylene chloride is cooled to below -60°C. A solution of 0.5 mL of dimethylsulfoxide in 1.5 mL of methylene chloride is added drop-wise and is maintained for 15 minutes at -60°C. 1 g of 1-[1-(4-fluorobenzyl)-1*H*-benzimidazol-2-yl]-4-hydroxypiperidine (3) in 3 mL of methylene chloride is added drop-wise. After half an hour at a low temperature, 2 mL of triethylamine are added.

It is heated to room temperature and 15 mL of water are added. The phases are decanted, and the aqueous phase is depleted with 7.5 mL of methylene chloride. The organic phases are brought together and the solvent is removed, obtaining 0.92 g of a solid corresponding to practically pure 1-[1-(4-fluorobenzyl)-1*H*-benzimidazol-2-yl]-4-piperidone (5).
¹³C-NMR (CDCl₃, δ in ppm): 40.92 (CH₂), 47.23 (CH₂), 50.50 (CH₂),122.28 (CH), 141.30 (C), 206.98 (C).

### EXAMPLE 6

### 1-[1-(4-Fluorobenzyl)-1H-benzimidazol-2-yl]-4-piperidone (5)

A mixture of 60 g of 8-[1-(4-fluorobenzyl)-1*H-*benzoimidazol-2-yl]-1,4-dioxa-8-azaspiro[4.5]decane (2) and 40 g of sulfuric acid dissolved in 240 mL of water and 300 mL of tetrahydrofuran is heated under reflux temperature for five hours. Once the hydrolysis is finished, the reaction mixture is cooled to room temperature and neutralized with sodium bicarbonate. 240 mL of water are added and the phases are decanted. The tetrahydrofuran is removed from the organic phase and the residue formed is dissolved in 540 mL of ethyl acetate and 640 mL of water with stirring, the organic phase is decanted and the solvent is removed to dryness to obtain 40 g of 1-[1-(4-fluorobenzyl)-1*H*-benzimidazol-2-yl]-4-piperidone (5).

### EXAMPLE 7

### 1-[1-(4-Fluorobenzyl)-1H-benzimidazol-2-yl]-N-methyl-4-iminopiperidine (6)

27 g of 1-[1-(4-fluorobenzyl)-1*H*-benzimidazol-2-yl]-4-piperidone (5) are dissolved in 135 mL of methylamine in methanol (7.5 M) at room temperature, after a given time, precipitating a white solid which is filtered and washed with methanol. 25 g of crude 1-[1-(4-fluorobenzyl)-1*H*-benzimidazol-2-yl]-N-methyl-4-iminopiperidine (6) are thus obtained.
¹³C-NMR (CDCl₃, δ in ppm): 38.16 (CH₃), 51.53 (CH₂), 141.48 (C), 169.14 (C).

### EXAMPLE 8

### 1-[1-(4-Fluorobenzyl)-1H-benzimidazol-2-yl]-N-methyl-4-aminopiperidine

40 g of 1-[1-(4-fluorobenzyl)-1*H*-benzimidazol-2-yl]-4-piperidone (5) are dissolved in 200 mL of methylamine in methanol (7.5 M) at room temperature, precipitating a white solid (the corresponding imine formed), after a given time. Half an hour after the imine has precipitated, it is considered that the first reaction has concluded, therefore, 4.7 g of sodium borohydride are then added. Once the reaction has concluded, it is processed by adding 200 mL of water and removing the methanol under reduced pressure. Then, 240 mL of methylene chloride are added and the pH is adjusted to 3 with hydrochloric acid.

The organic phase which is discarded is decanted, and another 120 mL of methylene chloride are added to the aqueous phase, and the pH is raised to 8.5 with a 50% sodium hydroxide solution. The organic phase is decanted and the aqueous phase is again extracted with another 60 mL of methylene chloride. The last two organic phases are brought together and the solvent is removed under reduced pressure to obtain 35 g of 1-[1-(4-fluorobenzyl)-1*H*-benzimidazol-2-yl]-N-methyl-4-aminopiperidine.
¹H-NMR (CDCl₃, δ in ppm): 1.6 (c), 2.0 (d), 2.5 (d), 2.7 (m), 3.0 (t), 3.5 (d), 5.2 (s), 7-7.6 (m).

### EXAMPLE 9

### 4-Benzyloxy-2-chloropyrimidine

A mixture formed by 18.9 g of sodium *tert-*butoxide and 40.6 g of benzyl alcohol in 80 mL of THF, is heated under reflux temperature for half an hour, is subsequently cooled to 0°C and slowly added drop-wise to 26.6 g of 2,4-dichloropyrimidine dissolved in 133 mL of N,N-dimethylformamide, maintaining the temperature below -50°C. After stirring for one hour, it is left to reach room temperature, the tetrahydrofuran is removed under reduced pressure and the obtained residue is added drop-wise to 800 mL of cold water, obtaining a precipitate which is dried in an oven with forced air circulation at a temperature of 60°C, once dried, it is resuspended for two hours in 78 mL of methanol, filtered and again dried in an oven with forced air circulation at a temperature of 60°C. 24 g of impurity free 4-benzyloxy-2-chloropyrimidine are obtained.
¹³C-NMR (CDCl₃, δ in ppm): 69.13 (CH₂), 107.31 (CH), 128.50 (CH), 135.11 (C), 160.11 (C).

### EXAMPLE 10

### 2-({1-[1-(4-Fluorobenzyl)-1H-benzimidazol-2-yl]-piperidin-4-yl}-aminomethyl)-4-benzyloxypyrimidine (7)

A mixture formed by 6.4 g of 1-[1-(4-fluorobenzyl)-1*H-*benzimidazol-2-yl]-N-methyl-4-aminopiperidine (9), 5 g of 4-benzyloxy-2-chloropyrimidine and 5.2 g of potassium carbonate in 64 mL of triethylamine is heated under reflux temperature for 62 hours. Once the reaction is finished, it is processed by removing the solvent under reduced pressure to obtain a residue, which is dissolved in 64 mL of methylene chloride and 62 mL of water. The mixture formed is neutralized with hydrochloric acid, the organic phase is separated and the aqueous phase is again extracted with 32 mL of methylene chloride. The organic phases are brought together, washed with 60 mL of water and the solvent is removed. The residue obtained is recrystallized in 64 mL of acetonitrile, obtaining 5.6 g of 2-({1-[1-(4-fluorobenzyl)-1*H*-benzimidazol-2-yl]-piperidin-4-yl}-aminomethyl)-4-benzyloxy-pyrimidine (7).
¹³C-NMR (CDCl₃, δ in ppm): 28.87 (CH₂), 29.03 (CH₃), 47.09 (CH₂), 67.04 (CH₂), 122.02 (CH), 141.61 (C), 169.04 (C).

### EXAMPLE 11

### 2-({1-[1-(4-fluorobenzyl)-1H-benzimidazol-2-yl]-piperidin-4-yl}-methylamino)-4(1H) pyrimidinone (mizolastine) (1)

A mixture formed by 5.6 g of 2-({1-[1-(4-fluorobenzyl)-1*H*-benzimidazol-2-yl]-piperidin-4-yl}-aminomethyl)-4-benzyloxy-pyrimidine (7) in 84 mL of methanol and 56 mL of hydrobromic acid in acetic acid is heated under reflux temperature for 12 hours. Once the reaction has finished, it is cooled and taken to pH 8 using a 50% sodium hydroxide solution. The methanol is removed under reduced pressure and 56 mL of methylene chloride and 56 mL of water are added, and it is stirred. Two phases are formed, the organic phase is separated and the aqueous phase is again extracted with another 28 mL of methylene chloride. The organic phases are washed separately with 56 mL of water, they are brought together and the solvent is removed. The residue obtained is resuspended in 56 mL of ethanol and filtered to give 2.4 g of crude 2-({1-[1-(4-fluorobenzyl)-1*H*-benzimidazol-2-yl]-piperidin-4-yl}-methylamino)-4(1*H*) pyrimidinone (1).
¹³C-NMR (CDCl₃, δ in ppm): 28.73 (CH₂), 29.03 (CH₃), 47.16 (CH₂), 52.68 (CH), 122.04 (CH), 135.26 (C), 141.61 (C), 165.99 (C).

## Claims

1. A compound of Formula I 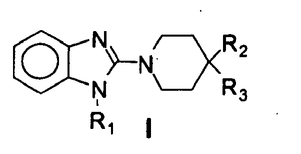 wherein:
R₁ is hydrogen or 4-halobenzyl;
one of R₂ or R₃ is hydrogen and the other one is OH, optionally protected with a protecting group of the hydroxyl group; or
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O- (CH₂)ₙ-O-, wherein n is a whole number comprised between 2 and 5, optionally substituted with one or more substituents selected from OH, C₁-C₅ alkyl, C₁-C₅ alkoxy and C₁-C₅ hydroxyalkyl.

2. A compound according to claim 1, wherein R₁ is hydrogen or 4-halobenzyl; R₂ is hydrogen; and R₃ is OH.

3. A compound according to claim 1, wherein:
R₁ is hydrogen or 4-halobenzyl; and
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy or C₁-C₅ hydroxyalkyl.

4. A compound according to claim 1, wherein R₁ is hydrogen or 4-halobenzyl; R₂ is hydrogen; and R₃ is OH protected with a protecting group of the hydroxyl group.

5. A compound according to claim 1, selected from:
a compound of Formula I wherein R₁ is hydrogen or 4-fluorobenzyl; R₂ is hydrogen; and R₃ is OH;
a compound of Formula I wherein R₁ is hydrogen or 4-fluorobenzyl; and R₂ and R₃ are methoxy; and
a compound of Formula I wherein R₁ is hydrogen or 4-fluorobenzyl; and R₂ and R₃, taken together, form an ethylenedioxy group.

6. A compound according to claim 1, selected from:
8-[1-(4-fluorobenzyl)-1*H*-benzimidazol-2-yl]-1,4-dioxa-8-azaspiro [4,5]decane;
1-[1-(9-fluorobenzyl)-1*H*-benzimidazol-2-yl]-4-hydroxypiperidine; and
1-[1-(4-fluorobenzyl)-1*H*-benzimidazol-2-yl]-4-piperidone dimethylacetal.

7. A process for obtaining a compound of Formula I, according to claim 1, comprising reacting a chlorobenzimidazole of Formula II: 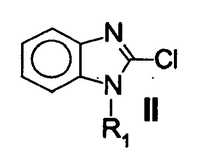 wherein R₁ is hydrogen or 4-halobenzyl;
with a piperidine of Formula III: 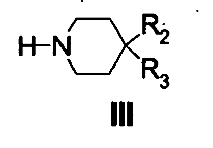 wherein
one of R₂ or R₃ is hydrogen and the other one is OH, optionally protected with a protecting group of the hydroxyl group; or
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy and C₁-C₅ hydroxyalkyl.

8. A process according to claim 7, further comprising converting a compound of Formula I wherein R₁ is hydrogen into a compound of Formula I wherein R₁ is 4-halobenzyl, by reacting with a 4-halobenzyl halide.

9. A process for obtaining a 1-(1*H*-benzimidazol-2-yl)-4-(2-aminopyrimidine)piperidine of Formula IV 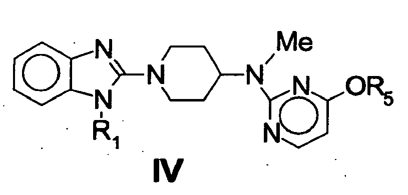 wherein:
R₁ is hydrogen or 4-halobenzyl; and
R₅ is hydrogen; C₁-C₅ alkyl optionally substituted with one or more substituents selected from halogen, nitro and an - OR₆ group, wherein R₆ is C₁-C₅ alkyl or benzyl; or benzyl optionally substituted in the aromatic ring with one or more substituents selected from halogen, nitro and an -OR₆ group, wherein R₆ is C₁-C₅ alkyl or benzyl;
comprising
a) converting a compound of Formula I, according to claim 1, into a 1-[1-(R₁)-1*H*-benzimidazol-2-yl]-4-piperidone of Formula V 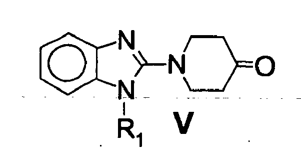 wherein R₁ has the previously mentioned meanings,
by hydrolysis and/or by oxidation of said compound of Formula I;
b) converting said compound of Formula V into a compound of Formula VI 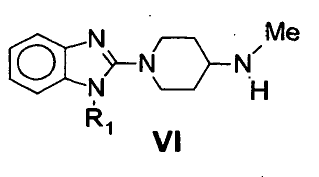 wherein R₁ has the previously mentioned meanings;
by reductive amination of said compound of Formula V with methylamine and reducing agent, with optional isolation of the intermediate imine formed;
c) reacting said compound of Formula VI with a compound of Formula VII 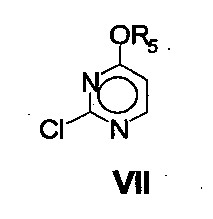 wherein R₅ has the previously mentioned meaning;
to obtain a compound of Formula IV; and, optionally
d) converting a compound of Formula IV wherein R₅ is different from hydrogen into a compound of Formula IV wherein R₅ is hydrogen.

10. A process according to claim 9, comprising the oxidation of a compound of Formula I wherein R₁ is hydrogen or 4-halobenzyl; R₂ is hydrogen; and R₃ is OH, to obtain said compound of Formula V.

11. A process according to claim 9, comprising the acid hydrolysis of a compound of Formula I wherein
R₁ is hydrogen or 4-halobenzyl; and
R₂ and R₃, the same or different, are C₁-C₅ alkoxy, optionally substituted with one or more substituents selected from OH, C₁-C₅ alkyl, C₁-C₅ alkoxy and C₁-C₅ hydroxyalkyl, or benzyloxy optionally substituted in the aromatic ring with one or more substituents selected from nitro, halogen, OH and C₁-C₅ alkoxy; or
R₂ and R₃, taken together, form a divalent group of formula -O-(CH₂)ₙ-O-, wherein n is a whole number comprised between 2 and 5, optionally substituted with one or more substituents selected from OH, C₁-C₅ alkyl, C₁-C₅ alkoxy or C₁-C₅ hydroxyalkyl
to obtain said compound of Formula V.

12. A process according to claim 9, comprising the hydrolysis of a compound of Formula I wherein R₁ is hydrogen or 4-halobenzyl; R₂ is hydrogen; and R₃ is OH protected with a protecting group of the hydroxyl group; and the subsequent oxidation of the hydroxylated intermediate generated, to obtain said compound of Formula V.

13. A process according to claim 9, comprising the isolation of the intermediate imine formed by reacting the compound of Formula V with methylamine.

14. A process according to claim 9, wherein the reducing agent used in step b) is sodium borohydride or sodium cyanoborohydride.

15. A process according to claim 9, wherein the reaction between the compound of Formula VI and the compound of Formula VII is carried out in the presence of an organic base, optionally accompanied by an inorganic base.

16. A process according to claim 9, wherein the obtained compound of Formula IV is 2-({1-[1-(4-fluorobenzyl)-1*H-*benzimidazol-2-yl]-piperidin-4-yl}-methylamino)-4(1*H*)pyrimidinone [mizolastine].

17. A process according to claim 9, wherein the obtained compound of Formula IV is 2-({1-[1-(4-fluorobenzyl)-1*H-*benzimidazol-2-yl]-piperidin-4-yl}-methylamino)-4-benzyloxypyrimidine.

18. A process according to claim 9, wherein the obtained compound of Formula IV is 2-({1-[1-(4-fluorobenzyl)-1*H-*benzimidazol-2-yl]-piperidin-4-yl}-methylamino)-4-benzyloxypyrimidine and further comprises the hydrolysis in acid medium of said compound to obtain 2-({1-[1-(4-fluorobenzyl)-1*H-*benzimidazol-2-yl]-piperidin-4-yl}-methylamino)-4(1*H*) pyrimidinone [mizolastine].

19. A compound of Formula V 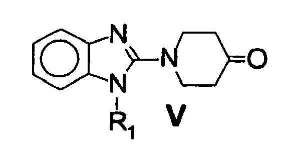 wherein R₁ is hydrogen or 4-halobenzyl.

20. A compound according to claim 19, **characterized in that** it is the compound 1-[1-(4-fluorobenzyl)-1*H*-benzimidazol-2-yl]-4-piperidone.

21. A compound of Formula VIII 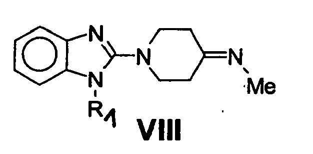 wherein R₁ is hydrogen or 4-halobenzyl.

22. A compound according to claim 21, **characterized in that** it is 1-[1-(4-fluorobenzyl)-1*H*-benzimidazol-2-yl]-N-methyl-4-iminopiperidine.

## Patentansprüche

1. Verbindung der Formel I 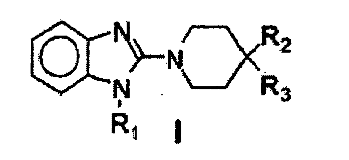 worin:
R₁ Wasserstoff oder 4-Halobenzyl ist;
einer von R₂ oder R₃ Wasserstoff ist und der andere OH, optional mit einer Schutzgruppe für die Hydroxylgruppe geschützt; oder
R₂ und R₃, gleich oder verschieden, C₁-C₅ Alkoxy sind, optional substituiert mit einem oder mehreren Substituenten ausgewählt aus OH, C₁-C₅ Alkyl, C₁-C₅ Alkoxy und C₁-C₅ Hydroxyalkyl, oder Benzyloxy, optional am aromatischen Ring mit einem oder mehreren Substituenten ausgewählt aus Nitro, Halogen, OH und C₁-C₅ Alkoxy substituiert; oder
R₂ und R₃, zusammengenommen, eine divalente Gruppe der Formel -O-(CH₂)ₙ-O- bilden, worin n eine ganze Zahl zwischen 2 und 5 ist, optional substituiert mit einem oder mehreren Substituenten ausgewählt aus OH, C₁-C₅ Alkyl, C₁-C₅ Alkoxy und C₁-C₅ Hydroxyalkyl.

2. Verbindung nach Anspruch 1, worin R₁ Wasserstoff oder 4-Halobenzyl ist; R₂ Wasserstoff und R₃ OH.

3. Verbindung nach Anspruch 1, worin: R₁ Wasserstoff oder 4-Halobenzyl ist; und R₂ und R₃, gleich oder verschieden, C₁-C₅ Alkoxy sind, optional substituiert mit einem oder mehreren Substituenten ausgewählt aus OH, C₁-C₅ Alkyl, C₁-C₅ Alkoxy und C₁-C₅ Hydroxyalkyl, oder Benzyloxy, optional am aromatischen Ring mit einem oder mehreren Substituenten ausgewählt aus Nitro, Halogen, OH und C₁-C₅ Alkoxy substituiert; oder R₂ und R₃, zusammengenommen, eine divalente Gruppe der Formel -O-(CH₂)ₙ-O- bilden, worin n eine ganze Zahl zwischen 2 und 5 ist, optional substituiert mit einem oder mehreren Substituenten ausgewählt aus OH, C₁-C₅ Alkyl, C₁-C₅ Alkoxy und C₁-C₅ Hydroxyalkyl.

4. Verbindung nach Anspruch 1, worin R₁ Wasserstoff oder 4-Halobenzyl ist; R₂ Wasserstoff ist; und R₃ ist mit einer Hydroxylschutzgruppe geschütztes OH.

5. Verbindung nach Anspruch 1, ausgewählt aus:
einer Verbindung der Formel I, worin R₁ Wasserstoff oder 4-Fluorbenzyl ist; R₂ Wasserstoff ist; und R₃ OH ist;
einer Verbindung der Formel I, worin R₁ Wasserstoff oder 4-Fluorbenzyl ist; R₂ und R₃ Methoxy sind; und
einer Verbindung der Formel I, worin R₁ Wasserstoff oder 4-Fluorbenzyl ist; und R₂ und R₃, zusammengenommen, eine Ethylendioxygruppe bilden.

6. Verbindung nach Anspruch 1, ausgewählt aus:
8-[1-(4-Fluorbenzyl)-1*H*-benzimidazol-2-yl]-1,4-dioxa-8-azaspiro[4,5]dekan;
1-[1-(4-Fluorbenzyl)-1*H*-benzimidazol-2-yl]-4-hydroxypiperidin; und
1-[1-(4-Fluorbenzyl)-1*H*-benzimidazol-2-yl]-4-piperidondimethylacetal.

7. Verfahren zum Erhalt einer Verbindung der Formel I nach Anspruch 1, umfassend das Umsetzen eines Chlorbenzimidazols der Formel II: 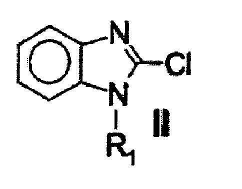 worin R₁ Wasserstoff oder 4-Halobenzyl ist; mit einem Piperidin der Formel III: 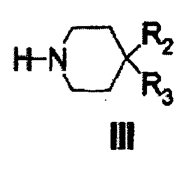 worin
einer von R₂ oder R₃ Wasserstoff ist und der andere OH, optional mit einer Hydroxylschutzgruppe geschützt; oder R₂ und R₃, gleich oder verschieden, C₁-C₅ Alkoxy sind, optional substituiert mit einem oder mehreren Substituenten ausgewählt aus OH, C₁-C₅ Alkyl, C₁-C₅ Alkoxy und C₁-C₅ Hydroxyalkyl, oder Benzyloxy, optional am aromatischen Ring mit einem oder mehreren Substituenten ausgewählt aus Nitro, Halogen, OH und C₁-C₅ Alkoxy substituiert; oder R₂ und R₃, zusammengenommen, eine divalente Gruppe der Formel -O-(CH₂)ₙ-O- bilden, worin n eine ganze Zahl zwischen 2 und 5 ist, optional substituiert mit einem oder mehreren Substituenten ausgewählt aus OH, C₁-C₅ Alkyl, C₁-C₅ Alkoxy und C₁-C₅ Hydroxyalkyl.

8. Verfahren gemäß Anspruch 7, ferner die Umwandlung einer Verbindung der Formel I, worin R₁ Wasserstoff ist, durch Umsetzen mit einem 4-Halobenzylhalogenid in eine Verbindung der Formel I, worin R₁ 4-Halobenzyl ist, umfassend.

9. Verfahren zum Erhalt eines 1-(1*H*-benzimidazol-2-yl)-4-(2-aminopyrimidin)piperidins der Formel IV 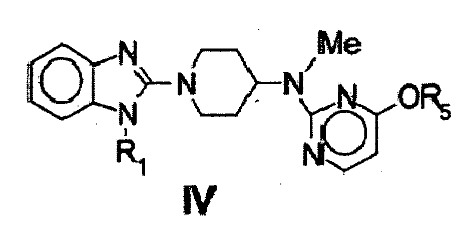 worin:
R₁ Wasserstoff oder 4-Halobenzyl ist; und R₅ Wasserstoff ist; C₁-C₅ Alkyl, optional substituiert mit einem oder mehreren Substituenten ausgewählt aus Halogen, Nitro und einer-OR₆-Gruppe, worin R₆ C₁-C₅ Alkyl oder Benzyl ist; oder optional mit einem oder mehreren Substituenten ausgewählt aus Halogen, Nitro und einer -OR₆-Gruppe, worin R₆ C₁-C₅ Alkyl oder Benzyl ist, am aromatischen Ring substituiertes Benzyl; umfassend
a) Umwandeln einer Verbindung der Formel I gemäß Anspruch 1 in ein 1-[1-(R₁)-1*H*-Benzimidazol-2-yl-]-4-piperidon der Formel V 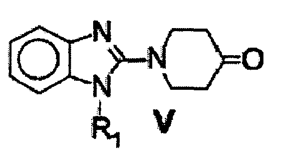 worin R₁ die vorgenannten Bedeutungen besitzt, durch Hydrolyse und/oder durch Oxidation der Verbindung der Formel I;
b) Umwandeln der Verbindung der Formel V in eine Verbindung der Formel VI 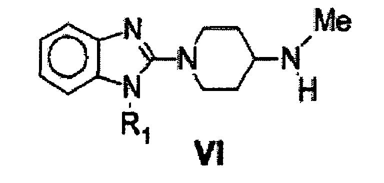 worin R₁ die vorgenannten Bedeutungen besitzt, durch reduktive Aminierung der Verbindung der Formel V mit Methylamin und Reduktionsmittel mit dem optionalen Isolieren des intermediär gebildeten Imins;
c) Umsetzen der Verbindung der Formel VI mit einer Verbindung der Formel VII 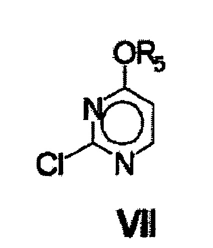 worin R₅ die vorgenannte Bedeutung besitzt; zum Erhalt einer Verbindung der Formel IV; und, optional,
d) Umwandeln einer Verbindung der Formel IV, worin R₅ von Wasserstoff verschieden ist, in eine Verbindung der Formel IV, worin R₅ Wasserstoff ist.

10. Verfahren gemäß Anspruch 9, umfassend die Oxidation einer Verbindung der Formel I, worin R₁ Wasserstoff oder 4-HAlobenzyl ist; R₂ Wasserstoff ist; und R₃ OH ist, zum Erhalt der Verbindung der Formel V.

11. Verfahren gemäß Anspruch 9, umfassend die sauere Hydrolyse einer Verbindung der Formel I, worin R₁ Wasserstoff oder 4-Halobenzyl ist; und R₂ und R₃, gleich oder verschieden, C₁-C₅ Alkoxy sind, optional substituiert mit einem oder mehreren Substituenten ausgewählt aus OH, C₁-C₅ Alkyl, C₁-C₅ Alkoxy und C₁-C₅ Hydroxyalkyl, oder Benzyloxy, optional am aromatischen Ring mit einem oder mehreren Substituenten ausgewählt aus Nitro, Halogen OH und C₁-C₅ Alkoxy substituiert; oder R₂ und R₃, zusammengenommen, eine divalente Gruppe der Formel -O-(CH₂)ₙ-O- bilden, worin n eine ganze Zahl zwischen 2 und 5 ist, optional substituiert mit einem oder mehreren Substituenten ausgewählt aus OH, C₁-C₅ Alkyl, C₁-C₅ Alkoxy und C₁-C₅ Hydroxyalkyl, um die Verbindung der Formel V zu erhalten.

12. Verfahren gemäß Anspruch 9, umfassend die Hydrolyse einer Verbindung der Formel I, worin R₁ Wasserstoff oder 4-Halobenzyl ist; R₂ Wasserstoff; und R₃ mit einer Hydroxylschutzgruppe geschütztes OH; und die nachfolgende Oxidation der erzeugten hydroxylierten intermediären Verbindung, zum Erhalt der Verbindung der Formel V.

13. Verfahren gemäß Anspruch 9, umfassend die Isolation des durch Umsetzen der Verbindung der Formel V mit Methylamin gebildeten intermediären Imins.

14. Verfahren gemäß Anspruch 9, bei dem das in Schritt b) verwendete Reduktionsmittel Natriumborhydrid oder Natriumcyanoborhydrid ist.

15. Verfahren gemäß Anspruch 9, bei dem die Umsetzung zwischen der Verbindung der Formel VI und der Verbindung der Formel VII in Gegenwart einer organischen Base durchgeführt wird, optional in Begleitung einer anorganischen Base.

16. Verfahren gemäß Anspruch 9, bei dem die erhaltene Verbindung der Formel IV 2-({1-[1-(4-Fluorbenzyl)-1*H*-benzimidazol-2-yl]-piperidin-4-yl}-methylamino)-4(1*H*)pyrimidinon (Mizolastin) ist.

17. Verfahren gemäß Anspruch 9, bei dem die erhaltene Verbindung der Formel IV 2-({1-[1-(4-Fluorbemyl)-1*H*-benzimidazol-2-yl]-piperidin-4-yl)-methylamino)-4-benzyloxypyrimidin ist.

18. Verfahren gemäß Anspruch 9, bei dem die erhaltene Verbindung der Formel IV 2-({1-[1-(4-Fluorbenzyl)-1*H*-benzimidazol-2-yl]-piperidin-4-yl}-methylamino)-4-benzyloxypyrimidin ist und ferner dieser Verbindung in acidem Medium zum Erhalt von 2-({1-[1-(4-Fluorbenzyl)-1*H*-benzimidazol-2-yl]-piperidin-4-yl}-methylamino)-4(1*H*)pyrimidinon (Mizolastin) umfasst.

19. Verbindung der Formel V 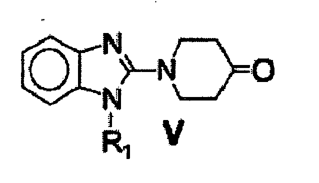 worin R₁ Wasserstoff oder 4-Halobenzyl ist.

20. Verbindung nach Anspruch 19, **dadurch gekennzeichnet, dass** es die Verbindung 1-[1-(4-Fluorbenzyl)-1*H*-benzimidazol-2-yl]-4-piperidon ist.

21. Verbindung der Formel VIII 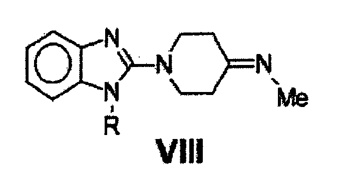 worin R₁ Wasserstoff oder 4-Halobenzyl ist.

22. Verbindung nach Anspruch 21, **dadurch gekennzeichnet, dass** es die Verbindung 1-[1-(4-Fluorbenzyl)-1*H*-benzimidazol-2-yl]-N-methyl-4-iminopiperidin ist.

## Revendications

1. Composé de formule I : 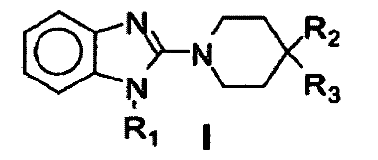 dans laquelle :
R₁ est l'hydrogène ou un radical 4-halogénobenzyle ;
l'un de R₂ ou R₃ est l' hydrogène et l'autre est OH, éventuellement protégé avec un groupe protecteur du groupe hydroxyle ; ou
R₂ et R₃, identiques ou différents, sont des radicaux alcoxy en C₁-C₅, éventuellement substitués par un ou plusieurs substituants choisis parmi les radicaux OH, alkyle en C₁-C₅, alcoxy en C₁-C₅ et hydroxyalkyle en C₁-C₅, ou benzyloxy éventuellement substitués sur le cycle aromatique par un ou plusieurs substituants choisis parmi les halogènes et les radicaux nitro, OH et alcoxy en C₁-C₅ ; ou
R₂ et R₃, pris ensemble, forment un groupe divalent de formule -O-(CH₂)ₙ-O- dans laquelle n est un nombre entier compris entre 2 et 5, éventuellement substitué par un ou plusieurs substituants choisis parmi les radicaux OH, alkyle en C₁-C₅, alcoxy en C₁-C₅ et hydroxyalkyle en C₁-C₅.

2. Composé selon la revendication 1, dans lequel R₁ est l'hydrogène ou un radical 4-halogénobenzyle ; R₂ est l'hydrogène ; et R₃ est OH.

3. Composé selon la revendication 1, dans lequel:
R₁ est l'hydrogène ou un radical 4-halogénobenzyle ;
R₂ et R₃, identiques ou différents, sont des radicaux alcoxy en C₁-C₅, éventuellement substitués par un ou plusieurs substituants choisis parmi les radicaux OH, alkyle en C₁-C₅, alcoxy en C₁-C₅ et hydroxyalkyle en C₁-C₅, ou benzyloxy éventuellement substitués sur le cycle aromatique par un ou plusieurs substituants choisis parmi les halogènes et les radicaux nitro, OH et alcoxy en C₁-C₅ ; ou
R₂ et R₃, pris ensemble, forment un groupe divalent de formule -O-(CH₂)ₙ-O- dans laquelle n est un nombre entier compris entre 2 et 5, éventuellement substitué par un ou plusieurs substituants choisis parmi les radicaux OH, alkyle en C₁-C₅, alcoxy en C₁-C₅ ou hydroxyalkyle en C₁-C₅.

4. Composé selon la revendication 1, dans lequel R₁ est l'hydrogène ou un radical 4-halogénobenzyle ; R₂ est l'hydrogène ; et R₃ est OH protégé avec un groupe protecteur du groupe hydroxyle.

5. Composé selon la revendication 1, choisi parmi:
un composé de formule I dans laquelle R₁ est l'hydrogène ou le radical 4-fluorobenzyle ; R₂ est l' hydrogène ; et R₃ est OH ;
un composé de formule I dans laquelle R₁ est l'hydrogène ou le radical 4-fluorobenzyle ; et R₂ et R₃ sont des radicaux méthoxy ; et
un composé de formule I dans laquelle R₁ est l'hydrogène ou le radical 4-fluorobenzyle ; et R₂ et R₃, pris ensemble, forment un groupe éthylènedioxy.

6. Composé selon la revendication 1, choisi parmi :
le 8-[1-(4-fluorobenzyl)-1*H*-benzimidazol-2-yl]-1,4-dioxa-8-azaspiro[4,5]décane ;
la 1-[1-(4-fluorobenzyl)-1*H*-benzimidazol-2-yl]-4-hydroxypipéridine ; et
le diméthylacétal de 1-[1-(4-fluorobenzyl)-1*H-*benzimidazol-2-yl]-4-pipéridone.

7. Procédé pour obtenir un composé de formule I, selon la revendication 1, comprenant la réaction d'un chlorobenzimidazole de formule II : 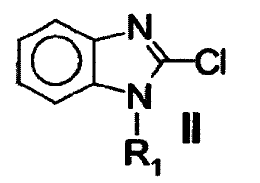 dans laquelle R₁ est l'hydrogène ou un radical 4-halogénobenzyle ;
avec une pipéridine de formule III : 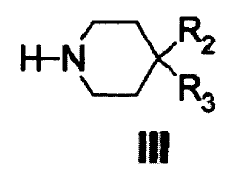 dans laquelle
l'un de R₂ et R₃ est l' hydrogène et l'autre est OH, éventuellement protégé avec un groupe protecteur du groupe hydroxyle ; ou
R₂ et R₃, identiques ou différents, sont des radicaux alcoxy en C₁-C₅, éventuellement substitués par un ou plusieurs substituants choisis parmi les radicaux OH, alkyle en C₁-C₅, alcoxy en C₁-C₅ et hydroxyalkyle en C₁-C₅, ou benzyloxy éventuellement substitués sur le cycle aromatique par un ou plusieurs substituants choisis parmi les halogènes et les radicaux nitro, OH et alcoxy en C₁-C₅ ; ou
R₂ et R₃, pris ensemble, forment un groupe divalent de formule -O-(CH₂)ₙ-O- dans laquelle n est un nombre entier compris entre 2 et 5, éventuellement substitué par un ou plusieurs substituants choisis parmi les radicaux OH, alkyle en C₁-C₅, alcoxy en C₁-C₅ et hydroxyalkyle en C₁-C₅.

8. Procédé selon la revendication 7, comprenant en outre la conversion d'un composé de formule I dans laquelle R₁ est l'hydrogène en un composé de formule I dans lequel R₁ est un radical 4-halogénobenzyle, par réaction avec un halogénure de 4-halogénobenzyle.

9. Procédé pour obtenir une 1-(1*H*-benzimidazol-2-yl)-4-(2-aminopyrimidine)pipéridine de formule IV : 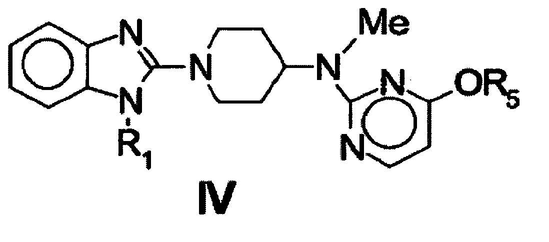 dans laquelle
R₁ est l'hydrogène ou un radical 4-halogénobenzyle ; et
R₅ est l'hydrogène ou un radical alkyle en C₁-C₅ éventuellement substitué par un ou plusieurs substituants choisis parmi les halogènes et les groupes nitro et -OR₆ où R₆ est un radical alkyle en C₁-C₅ ou benzyle ; ou benzyle éventuellement substitué sur le cycle aromatique par un ou plusieurs substituants choisis parmi les halogènes et les groupes nitro et -OR-6 où R₆ est un radical alkyle en C₁-C₅ ou benzyle ;
comprenant
a) la conversion d'un composé de formule I, selon la revendication 1, en une 1-[1-(R₁)-1*H*-benzimidazol-2-yl]-4-pipéridone de formule V : 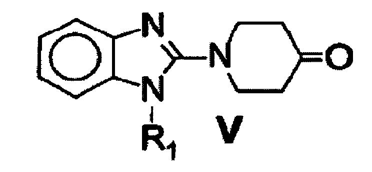 dans laquelle R₁ a les significations précédemment mentionnées ;
par hydrolyse et/ou par oxydation dudit composé de formule I ;
b) la conversion dudit composé de formule V en un composé de formule VI : 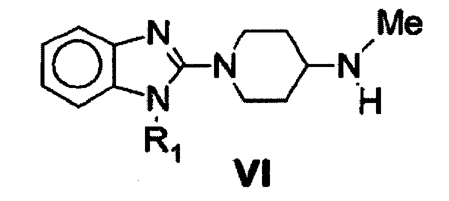 dans laquelle R₁ a les significations précédemment mentionnées ;
par amination réductrice dudit composé de formule V avec de la méthylamine et un agent réducteur, éventuellement avec isolation de l'imine intermédiaire formée ;
c) la réaction dudit composé de formule VI avec un composé de formule VII : 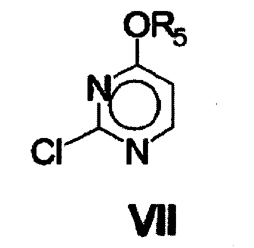 dans laquelle R₅ a les significations précédemment mentionnées ;
pour l'obtention d'un composé de formule IV ; et éventuellement
d) la conversion d'un composé de formule IV dans laquelle R₅ n'est pas l'hydrogène en un composé de formule IV dans laquelle R₅ est l' hydrogène.

10. Procédé selon la revendication 9, comprenant l'oxydation d'un composé de formule I dans laquelle R₁ est l'hydrogène ou un radical 4-halogénobenzyle ; R₂ est l'hydrogène ; et R₃ est OH, pour l'obtention dudit composé de formule V.

11. Procédé selon la revendication 9, comprenant l'hydrolyse acide d'un composé de formule I dans laquelle
R₁ est l'hydrogène ou un radical 4-halogénobenzyle ; et
R₂ et R₃, identiques ou différents, sont des radicaux alcoxy en C₁-C₅, éventuellement substitués par un ou plusieurs substituants choisis parmi les radicaux OH, alkyle en C₁-C₅, alcoxy en C₁-C₅ et hydroxyalkyle en C₁-C₅, ou benzyloxy éventuellement substitués sur le cycle aromatique par un ou plusieurs substituants choisis parmi les halogènes et les radicaux nitro, OH et alcoxy en C₁-C₅ ;ou
R₂ et R₃, pris ensemble, forment un groupe divalent de formule -O-(CH₂)ₙ-O- dans laquelle n est un nombre entier compris entre 2 et 5, éventuellement substitué par un ou plusieurs substituants choisis parmi les radicaux OH, alkyle en C₁-C₅, alcoxy en C₁-C₅ ou hydroxyalkyle en C₁-C₅, pour l'obtention dudit composé de formule V.

12. Procédé selon la revendication 9, comprenant l'hydrolyse d'un composé de formule I dans laquelle R₁ est l'hydrogène ou un radical 4-halogénobenzyle ; R₂ est l'hydrogène ; et R₃ est un radical OH protégé par un groupe protecteur du groupe hydroxyle ; et l'oxydation subséquente de l'intermédiaire hydroxylé généré, pour l'obtention dudit composé de formule V.

13. Procédé selon la revendication 9, comprenant l'isolation de l'imine intermédiaire formée par réaction du composé de formule V avec de la méthylamine.

14. Procédé selon la revendication 9, dans lequel l'agent réducteur utilisé dans l'étape b) est le borohydrure de sodium ou le cyanoborohydrure de sodium

15. Procédé selon la revendication 9, dans lequel la réaction entre le composé de formule VI et le composé de formule VII est mise en oeuvre en présence d'une base organique, éventuellement accompagnée d'une base inorganique.

16. Procédé selon la revendication 9, dans lequel le composé obtenu de formule IV est la 2-({1-[1-( 4-fluorobenzyl)-1*H*-benzimidazol-2-yl]-pipéridin-4-yl}méthylamino)-4(1*H*) pyrimidinone [mizolastine].

17. Procédé selon la revendication 9, dans lequel le composé obtenu de formule IV est la 2-({1-[1-(4-fluorobenzyl)-1*H*-benzimidazol-2-yl]-pipéridin-4-yl}méthylamino)-4-benzyloxypyrimidine.

18. Procédé selon la revendication 9, dans lequel le composé obtenu de formule IV est la 2-({1-[1-( 4-fluorobenzyl)-1*H*-benzimidazol-2-yl]-pipéridin-4-yl}méthylamino)-4-benzyloxypyrimidine et le procédé comprend en outre l'hydrolyse dans un milieu acide dudit composé pour l'obtention de 2-({1-[1-(4-fluorobenzyl)-1*H-*benzimidazol-2-yl]-pipéridin-4-yl}méthylamino)-4(1*H*) pyrimidinone [mizolastine].

19. Composé de formule V: 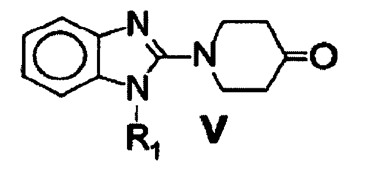 dans laquelle R₁ est l'hydrogène ou un radical 4-halogénobenzyle.

20. Composé selon la revendication 19, **caractérisé en ce qu'** il s' agit du composé 1-[1-(4-fluorobenzyl)-1*H-*benzimidazol-2-yl]-4-pipéridone.

21. Composé de formule VIII 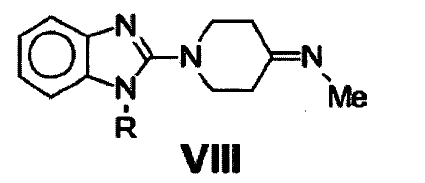 dans laquelle R₁ est l'hydrogène ou un radical 4-halogénobenzyle.

22. Composé selon la revendication 21, **caractérisé en ce qu'** il s'agit de la 1-[1-(4-fluorobenzyl)-1*H-*benzimidazol-2-yl]-N-méthyl-4-iminopipéridine.
